# EUROPEAN PATENT APPLICATION

(11) **EP 4 159 743 A1**
(43) Date of publication of application: **05.04.2023**
(21) Application number: 21200343.8
(22) Date of filing: 30.09.2021
(51) Int. Cl.: C07K 7/02, C07K 14/00, C12N 9/10, A61K 47/64

(54) **METHODS FOR PREPARING PYRIDAZINE COMPOUNDS**

(71) Applicant: ETH Zurich, 8092 Zurich (CH)
(72) Inventor: PIEL, Jörn, 8032 Zürich (CH); RICHTER, Daniel, 9487 Gamprin (CH); LAKIS, Edgars, 8050 Zürich (CH)

(57) **Abstract**

The present invention is directed to a method for preparing pyridazine compounds, optionally pyridazine comprising (poly)peptides, (poly)peptide derivatives and peptidomimetics, by reacting a compound, optionally a (poly)peptide, (poly)peptide derivative or peptidomimetic, comprising at least one α-keto-β³-glycine (aminopyruvate), with a non-, mono-, or di-substituted 1,2,4,5-tetrazine. It further relates to (poly)peptides, (poly)peptide derivatives and peptidomimetics comprising at least one pyridazine, the pyridazine optionally forming part of the (poly)-peptide and peptidomimetic backbone, in particular to said (poly)peptides, (poly)peptide derivatives and peptidomimetics further comprising an analytical, purification, diagnostic, medicinal, reactive, solubilization and/or stabilization substituent(s) bound to at least one pyridazine. The invention also encompasses corresponding uses of these (poly)peptides, (poly)peptide derivatives and peptidomimetics for identification, quantification, purification, solubilization and stabilization, therapy and/or diagnosis.

## Description

The present invention is directed to a method for preparing pyridazine compounds, optionally pyridazine comprising (poly)peptides, (poly)peptide derivatives and peptidomimetics, by reacting a compound, optionally a (poly)peptide, (poly)peptide derivative or peptidomimetic, comprising at least one α-keto-β³-glycine(aminopyruvate), with a non-, mono-, or di-substituted 1,2,4,5-tetrazine. It further relates to (poly)peptides, (poly)peptide derivatives and peptidomimetics comprising at least one pyridazine, the pyridazine optionally forming part of the (poly)-peptide and peptidomimetic backbone, in particular to said (poly)peptides, (poly)peptide derivatives and peptidomimetics further comprising an analytical, purification, diagnostic, medicinal, reactive, solubilization and/or stabilization substituent(s) bound to at least one pyridazine. The invention also encompasses corresponding uses of these (poly)peptides, (poly)peptide derivatives and peptidomimetics for identification, quantification, purification, solubilization and stabilization, therapy and/or diagnosis.

(Poly)peptide bioconjugates are highly relevant in the pharmaceutical industry, for example, for use as therapeutic antibody-drug conjugates, diagnostic radiolabeling conjugates, hapten-carrier vaccine conjugates and cross-linked protein complexes, to name just a few options. For the production of well-defined conjugates site-specific conjugation avoids unspecific functionalization.

Chemical reactions for imaging, detection, therapeutic and diagnostic purposes in living systems must be fast and bio-orthogonal. One of the most popular site-specific conjugation reactions, the dienophile-tetrazine ligation is limited by synthetic production, bulky size and incorporation of the dienophiles into the target biomolecules. Current approaches for introducing dienophiles in biorthogonal reactions generally suffer from shortcomings in terms of selectivity, low synthetic availability, low yields, low reactivity and low adduct stability.

Hoogenboom et al., J. Org. Chem., 2006, 71, 4903-4909 discloses the microwave-assisted synthesis of 3,6-di(pyridin-2-yl)pyridazines by means of ketone and aldehyde cycloadditions, in particular by inverse-electron-demand Diels-Alder reactions between enol tautomers of ketones and aldehydes and 1,2,4,5-tetrazines. These cycloadditions are generally slow and the effect of superheated microwave conditions thereon is described. Cycloaddition to 3,6-di(pyridin-2-yl)-1,2,4,5-tetrazine can be accelerated from several days reflux in toluene or *N,N*dimethylforma-mide to several hours in dichloromethane at 150 °C.

WO2018/125744 A1 discloses the use of a radical S-adenosyl methionine (rSAM) enzyme in a method for introducing at least one α-keto-β³-amino acid into (poly)peptide substrates comprising one or more of amino acid motif XYG, wherein X is any natural or non-natural amino acid, Y is tyrosine and G is glycine.

It is the objective of the present invention to provide new and improved methods for introducing functional substituents into compounds, in particular into (poly)peptides, optionally for identifying, purifying, labelling, diagnostic and/or therapeutic purposes of the compounds so prepared.

This objective is solved by a novel method for preparing pyridazine compounds, optionally pyridazine comprising (poly)peptides, (poly)peptide derivatives and peptidomimetics comprising the step of reacting
(i) a compound, optionally a (poly)peptide, (poly)peptide derivative or peptidomimetic comprising at least one α-keto-β³-glycine(aminopyruvate) with
(ii) a non-, mono-, or di-substituted 1,2,4,5-tetrazine
under conditions suitable for the formation of at least one pyridazine conjugate.

The methods of the present invention were demonstrated experimentally to be simple, robust, and site-specific and can be implemented under a great variety of conditions and environments. For example, they can be implemented *in vivo, ex vivo,* in many pure and mixed aqueous and organic solvents under highly varying reaction conditions and provide stable, in particular physiologically stable compounds suitable for *in vitro* and *in vivo* applications.

The conditions suitable for the formation of at least one pyridazine conjugate by the method of the present invention vary widely and may optionally be selected from the group consisting of aqueous compositions, pure water, organic compositions, alcoholic compositions, optionally pure methanol, aqueous buffered compositions, physiological compositions, phosphate-buffered saline (PBS), NPI-250 (50 mM sodium phosphate, 300 mM NaCl, 250 mM imidazole, pH 8), MES (200 mM MES, pH 5.5), *in vitro* and *in vivo* cellular conditions, *E. coli* lysate, *E. coli* cytosol, ambient temperatures, temperatures of 2 to 40, 5 to 37 °C, 15 to 37°, and a pH of 2 to 11, 4 to 10, 5 to 8.5.

The method of the present invention can be practiced with any compound comprising at least one α-keto-β³⁻glycine (aminopyruvate). And next to synthetically produced compounds such compounds may be (poly)peptides, polypeptide derivatives or peptidomimetics which can be conveniently produced by synthesis automation as well as recombinantly by cells and cell-related systems, rendering the compound suitable for easy and economic production.

The terms "(poly)peptide" and "(poly)peptide derivative", as used herein, are meant to encompass any natural amino acid sequence of at least 3 to 10 amino acids, and/or chemical derivative thereof, e.g. consisting of natural and/or non-natural amino acid equivalents, e.g. enzymatically or chemically derivatised at one or more functional groups, e.g. by glycosylation, PEGylation (PEG= poly(ethylene glycol) or poly(ethyleneoxide)) derivatisation, polyamide-PEG derivatisation, alkanoylation, carbamoylation, urea formation, including dimeric peptides, e.g. dimerisation via cysteine bonds, etc.

The term "peptidomimetic", as used herein, is meant to define small protein-like chains designed to mimic a peptide. They typically arise either from modification of an existing peptide, or by designing similar systems that mimic peptides, such as peptoids and β-peptides. The altered chemical structure is designed to advantageously adjust the molecular properties such as stability or biological activity. These modifications involve changes to the peptide that will not occur naturally (such as altered backbones and the incorporation of nonnatural amino acids). Based on their similarity with the precursor peptide, peptidomimetics can be grouped into four classes (A - D) where A, i.e. modified peptides, features the most and D, i.e. mechanistic mimetics, the least similarities. Classes A and B, i.e. modified peptides and peptidic foldamers involve peptide-like scaffolds, while classes C, i.e. structural mimetics including foldamers, and D include small molecules.

Therefore, in embodiments the method of the present invention further comprises the step of preparing the compound (i), wherein the compound is a (poly)peptide, polypeptide derivative or peptidomimetic comprising at least one α-keto-β³-glycine(aminopyruvate), by reacting
(a) (poly)peptides comprising one or more of amino acid motifs GYG, SYG, GYA, SYA, GFG, SFG, GFA, SFA, optionally one of amino acid motifs PIRPXYGLPI or AVAAXYGVVFP, wherein X is glycine or serine, with
(b) a radical S-adenosyl methionine (rSAM) enzyme that excises tyramine equivalent (C₈H₉ON) from at least one of the amino acid motifs in (a) to produce an α-keto-β³-glycine (aminopyruvate),
wherein the (poly)peptides and/or the rSAM enzyme are produced by cells expressing and optionally secreting the (poly)peptides and/or the rSAM enzyme, and
wherein the α-keto-β³-glycine(aminopyruvate) is produced within or outside the cells.

Optionally, the (poly)peptides, the (poly)peptide derivatives and the rSAM enzyme are expressed within the same cells. For example, when expressed in the same cells the (poly)peptides and the rSAM enzyme can react within the cell or they can be secreted and react outside the cells.

rSAM enzymes suitable for obtaining a (poly)peptide, (poly)peptide derivative or peptidomimetic comprising at least one α-keto-β³-glycine(aminopyruvate) for practicing the method of the present invention are widely available. For example, suitable cells and recombinant cells and rSAMs for producing these (poly)peptide and (poly)peptide derivative are disclosed and specifically listed by their aa and na sequences in WO2018/125744 A1. In further embodiments of the present invention the rSAM enzyme for practicing the method of the present invention is optionally selected from the group consisting of
(a) rSAM enzymes comprising, optionally identical to, one of amino acid sequences of SEQ ID NOs. 2 or 4;
(b) rSAM enzymes having an amino acid sequence identity of at least 70 or 80 %, optionally at least 90 or 95 %, with one of amino acid sequences of SEQ ID NOs. 2 and 4;
(c) rSAM enzymes that are functional fragments and/or functional derivatives of (a) or (b);
(d) rSAM enzymes encoded by a nucleic acid sequence of SEQ ID NOs: 1 or 3;
(e) rSAM enzymes encoded by a nucleic acid sequence of at least 80 or 90 % sequence identity, optionally at least 95 % or 98 % sequence identity, with one of SEQ ID NOs: 1 or 3, optionally over the whole sequence;
(e) rSAM enzymes encoded by a nucleic acid sequence that hybridizes to one of SEQ ID NOs: 1 or 3 under stringent conditions;
(f) rSAM enzymes encoded by a nucleic acid sequence degenerated with respect to a nucleic acid sequence listed in any of (c) to (e).

The percentage identity of related amino acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two amino acid sequences comprise, but are not limited to, TBLASTN, BLASTP, BLASTX, TBLASTX (Altschul et al., J. Mol. Biol., 215, 403-410, 1990), or ClustalW (Larkin MA et al., Bioinformatics, 23, 2947-2948, 2007). The BLAST programs can be obtained from the National Center for Biotechnology Information (NCBI) and from other sources (BLAST handbook, Altschul et al., NCB NLM NIH Bethesda, MD 20894). The ClustalW program can be obtained from http://www.clustal.org.

The term "functional derivative" of a (poly)peptide of the present invention is meant to include any (poly)peptide or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative still has at least some rSAM activity to a measurable extent, e.g. of at least about 1 to 10%, preferably 10 to 50% rSAM activity of the original unmodified (poly)peptide of the invention.

In this context a "functional fragment" of the invention is one that forms part of a (poly)-peptide or derivative of the invention and still has at least some rSAM activity to a measurable extent, e.g. of at least about 1 to 10%, preferably 10 to 50% rSAM activity of the original unmodified (poly)peptide of the invention.

The term nucleic acid sequence, as used herein, encompasses DNA, RNA and optionally PNA.

The term "nucleic acid that hybridizes with another nucleic acid", as used herein, for example hybridizing with another nucleic acid having a sequence of SEQ ID NOs 1 or 3, indicates a nucleic acid sequence that hybridizes under stringent conditions with a specified counterpart nucleic acid. For example, hybridizing may be performed and experimentally verified at 68°C in 2x SSC or according to the protocol of the dioxygenine-labeling-kits of the Boehringer (Mannheim) company. A further example of stringent hybridizing conditions is, for example, the incubation at 65°C overnight in 7% SDS, 1% BSA, 1mM EDTA, 250 mM sodium phosphate buffer (pH 7.2) and subsequent washing at 65°C with 2x SSC; 0.1% SDS.

The term "percent sequence identity" of nucleic acids, as known to the skilled artisan and used herein, indicates the degree of relatedness among 2 or more nucleic acid molecules that is determined by agreement among the sequences. The percentage of "sequence identity" is the result of the percentage of identical regions in 2 or more sequences while taking into consideration the gaps and other sequence peculiarities.

The identity of related nucleic acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Computer programs for determining the identity among two sequences comprise, but are not limited to, the GCG-program package, including GAP (Devereux et al., Nucleic Acids Research 12 (12):387 (1984); Genetics Computer Group University of Wisconsin, Madison, (WI)); BLASTP, BLASTN, and FASTA (Altschul et al., J. Molec. Biol 215:403/410 (1990)). The BLAST X program can be obtained from the National Center for Biotechnology Information (NCBI) and from other sources (BLAST handbook, Altschul et al., NCB NLM NIH Bethesda, MD 20894). Also, the well-known Smith-Waterman algorithm can be used for determining nucleic sequence identity.

Preferred parameters for nucleic sequence comparison comprise the following:

| Algorithm | Needleman and Wunsch, J. Mol. Biol. 48:443 - 453 (1970) |
|---|---|
| Comparison matrix | Matches =+10, mismatch 0 |
| Gap penalty: | 50 |
| Gap length penalty: | 3 |

The gap program is also suited to be used with the above-mentioned parameters. The above-mentioned parameters are standard parameters (default) for nucleic acid comparisons.

Further exemplary algorithms, gap opening penalties, gap extension penalties, comparison matrix, including those in the program handbook, Wisconsin-package, version 9, September 1997, can also be used. The selection depends on the comparison to be done and further, whether a comparison among sequence pairs, for which GAP or Best Fit is preferred, or whether a comparison among a sequence and a large sequence databank, for which FASTA or BLAST is preferred, is desired.

The term "degenerated nucleic acid sequence", as used herein, relates to the degeneracy or redundancy of codons in the genetic code. Hence, a nucleic acid sequence degenerated with respect to a further nucleic acid sequence codes for the same amino acid sequence but with one or more different codons that encode the same amino acid in the polypeptide sequence.

The method for preparing pyridazine compounds of the present invention can conveniently and under a wide variety of mild reaction conditions introduce mono- and disubstituted 1,2,4,5 tetrazines into compounds, optionally α-keto-β³-glycine comprising (poly)peptides, (poly)peptide derivatives and peptidomimetics. Therefore, the method is highly efficient, economical and mild for introducing substituents of diverse functionality into compounds, in particular into (poly)peptides, (poly)peptide derivatives and peptidomimetics forming part of mono- and disubstituted 1,2,4,5 tetrazines. For example, the functionality of the carbon 3 and/or carbon 6 substituent(s) on 1,2,4,5 tetrazines for practicing the method of the present invention can be analytical (e.g. identification, quantification, localization), for facilitating compound purification (e.g. binding to carriers, gels, beads, sedimentation, etc.), for diagnostic use (e.g. identifying, quantifying, localizing disease markers, e.g. cancer, Alzheimer plaques, infections, etc.), for medical use (i.e. for treating or preventing specific diseases), for reacting with specific targets (e.g. for fixating or modifying specific target compounds, e.g. biological structures), for solubilizing compounds (e.g. improving or reducing solubility of the pyridazine comprising compounds), or for stabilizing the pyridazine comprising compounds (e.g. for improving chemical or metabolic stability, for reducing metabolic degradation, etc.).

In embodiments, the method of the present invention is one, wherein the mono-, or disubstituted 1,2,4,5-tetrazines are selected from the group consisting of tetrazines, wherein the one or two carbon substituents in positions 3 and 6 are selected from the group consisting of (a) analytical substituents, optionally for identifying and/or quantifying poly(peptides), (b) purification substituents, optionally for at least partially purifying poly(peptides), (c) diagnostic substituents, (d) medicinal substituents, (e) reactive substituents, (f) solubilization substituents, and (g) stabilization substituents.

The tetrazine substituents for practicing the invention include many different compound classes and formats and encompass, for example, small and large organic compounds, (poly)peptides, peptidomimetics, antibodies, antibody fragments, diverse antibody formats, derivatives and mimetics, polymers, (poly)saccharides, PEGs, biotin, HA tag, His tag, glutathione, dyes, radiolabels, etc.

The method of the present invention is particularly suited for introducing antibodies, optionally monoclonal antibodies, functional fragments or functional derivatives thereof, or antibody-like binding proteins into pyridazine comprising compounds, optionally (poly)peptides, (poly)peptide derivatives or peptidomimetics.

Antibodies, functional fragments and functional derivatives thereof for practicing the method of the invention are routinely available by hybridoma technology (Kohler and Milstein, Nature 256, 495-497, 1975), antibody phage display (Winter et al., Annu. Rev. Immunol. 12, 433-455, 1994), ribosome display (Schaffitzel et al., J. Immunol. Methods, 231, 119-135, 1999) and iterative colony filter screening (Giovannoni et al., Nucleic Acids Res. 29, E27, 2001) once the target antigen is available. Typical proteases for fragmenting antibodies into functional products are well-known. Other fragmentation techniques can be used as well as long as the resulting fragment has a specific high affinity and, preferably a dissociation constant in the micromolar to picomolar range.

A very convenient antibody fragment for targeting applications is the single-chain Fv fragment, in which a variable heavy and a variable light domain are joined together by a polypeptide linker. Other antibody fragments for vascular targeting applications include Fab fragments, Fab2 fragments, miniantibodies (also called small immune proteins), tandem scFv-scFv fusions as well as scFv fusions with suitable domains (e.g. with the Fc portion of an immunoglobulin). For a review on certain antibody formats, see Holliger P, Hudson PJ.; Engineered antibody fragments and the rise of single domains. Nat Biotechnol. 2005 Sep., 23(9):1126-36.

The term "functional derivative" of an antibody for use in the method of the present invention is meant to include any antibody or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative has substantially the same binding affinity as to its original antigen and, preferably, has a dissociation constant in the micro-, nano- or picomolar range. A most preferred derivative of the antibodies for use in the present invention is an antibody fusion protein that will be defined in more detail below.

In embodiments, the antibody, fragment or functional derivative thereof for use in the method of the invention is one that is selected from the group consisting of polyclonal antibodies, monoclonal antibodies, chimeric antibodies, humanized antibodies, CDR-grafted antibodies, Fv-fragments, Fab-fragments and Fab2-fragments and antibody-like binding proteins, e.g. affilins, anticalins and aptamers.

For a review of antibody-like binding proteins see Binz et al. on engineering binding proteins from non-immunoglobulin domains in Nature Biotechnology, Vol. 23, No. 10, October 2005, 12571268. The term "aptamer" describes nucleic acids that bind to a polypeptide with high affinity. Aptamers can be isolated from a large pool of different single-stranded RNA molecules by selection methods such as SELEX (see, e.g., Jayasena, Clin. Chem., 45, p. 1628 - 1650, (1999); Klug and Famulok, M. Mol. Biol. Rep., 20, p. 97 -107 (1994); US 5,582,981). Aptamers can also be synthesized and selected in their mirror form, for example, as the L-ribonucleotide (Nolte et al., Nat. Biotechnol., 14, pp.1116 - 1119, (1996); Klussmann et al., Nat. Biotechnol., 14, p. 1112 - 1115, (1996)). Forms isolated in this way have the advantage that they are not degraded by naturally occurring ribonucleases and, therefore, have a greater stability.

Another antibody-like binding protein and alternative to classical antibodies are the so-called "protein scaffolds", for example, anticalins, that are based on lipocalin (Beste et al., Proc. Natl. Acad. Sci. USA, 96, p. 1898 - 1903, (1999)). The natural ligand binding sites of lipocalins, for example, of the retinol-binding protein or bilin-binding protein, can be changed, for example, by employing a "combinatorial protein design" approach, and in such a way that they bind selected haptens (Skerra, Biochem. Biophys. Acta, 1482, pp. 337 - 350, (2000)). For other protein scaffolds it is also known that they are alternatives for antibodies (Skerra, J. Mol. Recognit, 13, pp. 167 - 287, (2000)). (Hey, Trends in Biotechnology, 23, pp. 514-522, (2005)).

In representative and non-limiting embodiments, the one or two carbon substituents in positions 3 and 6 of the mono-, or di-substituted 1,2,4,5-tetrazines for practicing the present invention are
(a) analytical substituents selected from the group consisting of
   (i) fluorescent molecules, optionally selected from the group consisting of fluorescent dyes, optionally Alexa Fluor^{®} 350, 405, 430, 488, 532, 546, 555, 568, 594, 595, 610, 633, 635, 647, 660, 680, 700, 750, 790;
   (ii) Forster Resonance Energy Transfer (FRET)-labels, optionally at least one member of a FRET pair and including at least one member of a fluorophore/quencher pair;
   (iii) Bioluminescence Resonance Energy Transfer (BRET)-labels, optionally at least one member of a BRET pair;
   (iv) radioactive substituents, optionally selected from the group consisting of ligands for ¹¹¹In, ¹²⁵I, ¹³¹I, ²¹²B, ⁹⁰Y, ¹⁸⁶Rh;
   (v) recognition tags, optionally immunodetectable tags, antibodies, fragments, derivatives and analogs thereof, FLAG (Hopp, Biotechnology, 6, pp. 1204-1210, (1998)), His(6), influenza hemagglutinin (HA) tag;
(b) purification substituents selected from the group consisting of biotin, peptide substituents, optionally His(6), HA, FLAG, optionally attached to a solid substrate, optionally beads, magnetic beads, agarose polymeric gel, chips;
(c) diagnostic substituents selected from the group consisting of
   (i) radiolabels, optionally ligands for ¹¹¹In, ¹²⁵I, ¹³¹I, ²¹²B, ⁹⁰Y, ¹⁸⁶Rh, and
   (ii) near-IR fluorescent dyes, optionally Alexa Fluor^{®} 750, 790;
(d) medicinal substituents selected from the group consisting of
   (i) cancer chemotherapeutic agents, alkylating agents, alkaloids, dolastatin, auristatins, maytansinoids;
   (ii) cellular proliferation reducing agents, optionally duocarmycins, mechlorethamine, cyclophosphamide (Cytoxan^{™}), melphalan (L-sarcolysin), carmustine (BCNU), lomustine (CCNU), semustine (methyl-CCNU), streptozocin, chlorozotocin, uracil mustard, chlormethine, ifosfamide, chlorambucil, pipobroman, triethylenemelamine, triethylenethiophosphoramine, busulfan, dacarbazine, and temozolomide;
   (iii) antimetabolite agents, optionally cytarabine (CYTOSAR-U), cytosine arabinoside, fluorouracil (5-FU), floxuridine (FudR), 6-thioguanine, 6-mercaptopurine (6-MP), pentostatin, 5-fluorouracil (5-FU), methotrexate, 10-propargyl-5,8- dideazafolate (PDDF, CB3717), 5,8-dideazatetrahydrofolic acid (DDATHF), leucovorin, fludarabine phosphate, pentostatine, gemcitabine;
   (iv) cytotoxic natural products, optionally Ara-C, paclitaxel (Taxol^{®}), docetaxel (Taxotere^{®}), deoxycoformycin, mitomycin-C, L-asparaginase, azathioprine, brequinar, vincristine, vinblastine, vinorelbine, vindesine, podophyllotoxins, etoposide, teniposide, antibiotics, anthracycline, daunorubicin, rubidomycin, cerubidine, idarubicin, doxorubicin, epirubicin, phenoxizone biscyclopeptides, dactinomycin, bleomycin, plicamycin (mithramycin), mitoxantrone, mitomycin, calicheamicin;
   (v) macrocyclic immunosuppressants, optionally cyclosporine, FK-506 (tacrolimus, prograf), rapamycin;
   (vi) microtubule affecting agents, optionally allocolchicine (NSC 406042), Halichondrin B (NSC 609395), colchicine (NSC 757), colchicine derivatives, optionally NSC 33410, dolstatin 10 (NSC 376128), maytansine (NSC 153858), rhizoxin (NSC 332598), paclitaxel (Taxol^{®}), Taxol^{®} derivatives, docetaxel (Taxotere^{®}), thiocolchicine (NSC 361792), trityl cysterin, vinblastine sulfate, vincristine sulfate, natural and synthetic, discodermolide; estramustine, nocodazole;
   (vii) hormone modulators and steroids, optionally adrenocorticosteroids, optionally prednisone, dexamethasone, estrogens and progestins, optionally hydroxyprogesterone caproate, medroxyprogesterone acetate, megestrol acetate, estradiol, clomiphene, tamoxifen; and adrenocortical suppressants, optionally aminoglutethimide, 17α-ethinylestradiol, diethylstilbestrol, testosterone, fluoxymesterone, dromostanolone propionate, testolactone, methylprednisolone, methyl-testosterone, prednisolone, triamcinolone, chlorotrianisene, hydroxyprogesterone, aminoglutethimide, estramustine, medroxyprogesterone acetate, leuprolide, Flutamide (Drogenil), Toremifene (Fareston), Zoladex^{®};
   (viii) immunosuppressants, optionally mycophenolic acid, thalidomide, desoxyspergualin, azasporine, leflunomide, mizoribine, azaspirane (SKF 105685), Iressa^{®} (ZD 1839, 4-(3-chloro-4-fluorophenylamino)-7-methoxy-6-(3-(4-morpholinyl)propoxy)quinazoline); and
   (ix) cisplatin (cis-DDP), carboplatin, hydroxyurea, N-methylhydrazine; epidophyllotoxin, procarbazine, mitoxantrone, leucovorin, tegafur;
(e) reactive substituents, optionally selected from the group consisting of alkynes, trans-cyclooctene, norbornene, aldehyde, tetrazine, azides, difluorooctyne, nitrones, cyclopropene;
(f) solubilization substituents selected from the group consisting of biological polymers, optionally dextran, dextran derivative, cellulose, methylcellulose, starch, dextrins, sialic acids, and non-natural polymers, optionally polyethylene glycol (PEG), polyvinyl ethyl ethers, polyvinylpyrrolidone, polyoxyethylated polyols, polyalkylene oxides, polyamide alkylene oxides; and
(g) stabilization substituents, optionally selected from the herein mentioned substituents, and providing a stabilization effect, and small organic substituents, optionally linear or branched, substituted or non-substituted C1 to C10 alkyl, alkenyl, hydrogen, C3 to C10 cyclyl, and heterocyclyl.
Reactive substituents are suitable for reaction and conjugation.

In further embodiments, the mono-, or di-substituted 1,2,4,5-tetrazine for practicing the method of the present invention may be selected tetrazines, wherein the one or two carbon substituents in positions 3 and 6 are independently selected from the group consisting of hydrogen, halogen, optionally CI, Br, and F, linear or branched, substituted and non-substituted alkyl, alkenyl, alkynyl, alkoxy, amino, carboxyl, carboxyl ester, acyl, acyloxy, acyl amino, amino acyl, alkylamide, sulfonyl, thioalkoxy, aryl, and heteroaryl, cycloalkyl, and heterocyclyl, (poly)peptides, (poly)peptide derivatives, peptidometics, and mono-, di-, tri- and polysaccharides.

The above terms "linear or branched, substituted or non-substituted have well defined meanings in organic chemistry and refer to all of the following compound classes.

As used herein, the term "substituent" refers to a molecular moiety that is covalently bound to an atom within a molecule of interest. For example, a "substituent" may be a moiety such as a halogen, alkyl group, haloalkyl group or any other substituent described herein that is covalently bonded to an atom, optionally a carbon or nitrogen atom, that forms part of a molecule of interest.

The term "heteroatom" as used herein shall be understood to mean atoms other than carbon and hydrogen such as O, N, S and P.

"Halogen" encompasses fluoro, chloro, bromo, and iodo.

"Alkyl" refers to linear or branched saturated aliphatic hydrocarbyl groups of optionally 1 to 10, optionally 1 to 6 carbon atoms, e.g. methyl, ethyl, n-propyl, isopropyl, n-butyl, isobutyl, sec-butyl, t-butyl, n-pentyl, and neopentyl, etc. "Substituted alkyl" refers to linear or branched alkyl groups, wherein one or more carbon atoms in the alkyl chain have been optionally replaced with a heteroatom, e.g. -O-, -N-, -S-, -S(O)n- (where n is 0 to 2), -NR- (where R is hydrogen or alkyl), and optionally having from 1 to 5 substituents, optionally selected from the group consisting of substituted or non-substituted alkoxy, cycloalkyl, cycloalkenyl, cycloalkenyl, acyl, acylamino, acyloxy, amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy,thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-aryl, -SO-heteroaryl, -SO2-alkyl, -S02-aryl, -SO2-heteroaryl, and -NRaRb, wherein Ra and Rb" may be the same or different and are chosen from hydrogen, optionally substituted alkyl, cycloalkyl, alkenyl, cycloalkenyl, alkynyl, aryl, heteroaryl and heterocyclic.

"Alkenyl" refers to linear or branched hydrocarbyl groups, optionally with 2 to 6 or 2 to 4 carbon atoms and having at least 1 or more, optionally 1 to 2 sites of double bond unsaturation, e.g. bivinyl, allyl, and but-3-en-1-yl. The term includes cis and trans isomers or mixtures thereof. The term "substituted alkenyl" refers to an alkenyl group, optionally having 1 to 5 or 1 to 3 substituents, optionally selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-substituted alkyl, -SO-aryl, -SO-heteroaryl, -SO2-alkyl, -SO2-substituted alkyl, -SO2-aryl and - SO2-heteroaryl.

"Alkynyl" refers to linear or branched monovalent hydrocarbyl groups, optionally having 2 to 6 or 2 to 3 carbon atoms and having at least 1 or more and optionally 1 to 2 sites of triple bond unsaturation. Examples of such alkynyl groups include acetylenyl (-C=CH), and propargyl (-CH2C=CH). The term "substituted alkynyl" refers to an alkynyl group, optionally having 1 to 5 or 1 to 3 substituents, optionally selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-substituted alkyl, -SO-aryl, -SO-heteroaryl, -SO2-alkyl, -SO2-substituted alkyl, -SO2-aryl, and-SO2-heteroaryl.

"Alkoxy" includes, by way of example, methoxy, ethoxy, n-propoxy, isopropoxy, n-butoxy, t-butoxy, sec-butoxy, n-pentoxy, and the like. The term "alkoxy" also refers to the groups alkenyl-O-, cycloalkyl-O-, cycloalkenyl-O-, and alkynyl-O-, where alkenyl, cycloalkyl, cycloalkenyl, and alkynyl are as defined herein. The term "substituted alkoxy" includes substituted alkyl-O-, substituted alkenyl-O-, substituted cycloalkyl-O-, substituted cycloalkenyl-O-, and substituted alkynyl-O-, where substituted alkyl, substituted alkenyl, substituted cycloalkyl, substituted cycloalkenyl and substituted alkynyl are as defined herein.

"Aryl" refers to an aromatic carbocyclic group of 6 to 18 carbon atoms having a single ring (such as is present in a phenyl group) or a ring system having multiple condensed rings (examples of such aromatic ring systems include naphthyl, anthryl and indanyl) which condensed rings may or may not be aromatic, provided that the point of attachment is through an atom of an aromatic ring. For example, aryl groups can optionally be substituted with from 1 to 5 substituents, or from 1 to 3 substituents, selected from acyloxy, hydroxy, thiol, acyl, alkyl, alkoxy, alkenyl, alkynyl, cycloalkyl, cycloalkenyl, substituted alkyl, substituted alkoxy, substituted alkenyl, substituted alkynyl, substituted cycloalkyl, substituted cycloalkenyl, amino, substituted amino, aminoacyl, acylamino, alkaryl, aryl, aryloxy, azido, carboxyl, carboxylalkyl, cyano, halogen, nitro, heteroaryl, heteroaryloxy, heterocyclyl, heterocyclooxy, aminoacyloxy, oxyacylamino, thioalkoxy, substituted thioalkoxy, thioaryloxy, thioheteroaryloxy, -SO-alkyl, -SO-substituted alkyl, -SO-aryl, -SO-heteroaryl, -SO2-alkyl, -SO2-substituted alkyl, -S02-aryl, -SO2-heteroaryl and trihalomethyl. "Heteroaryl" refers to an aromatic group of from 1 to 15 carbon atoms, such as from 1 to 10 carbon atoms and 1 to 10 heteroatoms selected from the group consisting of oxygen, nitrogen, and sulfur within the ring. Such heteroaryl groups can have a single ring (such as, pyridinyl, imidazolyl or furyl) or multiple condensed rings in a ring system (for example as in groups such as, indolizinyl, quinolinyl, benzofuran, benzimidazolyl or benzothienyl), wherein at least one ring within the ring system is aromatic and at least one ring within the ring system is aromatic, provided that the point of attachment is through an atom of an aromatic ring. In certain embodiments, the nitrogen and/or sulfur ring atom(s) of the heteroaryl group are optionally oxidized to provide for the N-oxide (N→O), sulfinyl, or sulfonyl moieties. This term includes, by way of example, pyridinyl, pyrrolyl, indolyl, thiophenyl, and furanyl. Such heteroaryl groups can be optionally substituted with 1 to 5 substituents, or from 1 to 3 substituents, selected from acyloxy, hydroxy, thiol, acyl, alkyl, alkoxy, alkenyl, alkynyl, cycloalkyl, cycloalkenyl, substituted alkyl, substituted alkoxy, substituted alkenyl, substituted alkynyl, substituted cycloalkyl, substituted cycloalkenyl, amino, substituted amino, aminoacyl, acylamino, alkaryl, aryl, aryloxy, azido, carboxyl, carboxylalkyl, cyano, halogen, nitro, heteroaryl, heteroaryloxy, heterocyclyl, heterocyclooxy, aminoacyloxy, oxyacylamino, thioalkoxy, substituted thioalkoxy, thioaryloxy, thioheteroaryloxy, -SO-alkyl, -SO-substituted alkyl, -SO-aryl, -SO-heteroaryl, -SO2-alkyl, -SO2-substituted alkyl, -SO2-aryl and -SO2-heteroaryl, and trihalomethyl.

"Cycloalkyl" refers to cyclic alkyl groups of from 3 to 10 carbon atoms having single or multiple cyclic rings including fused, bridged, and spiro ring systems. Examples of suitable cycloalkyl groups include, for instance, adamantyl, cyclopropyl, cyclobutyl, cyclopentyl, cyclooctyl and the like. Such cycloalkyl groups include, by way of example, single ring structures such as cyclopropyl, cyclobutyl, cyclopentyl, cyclooctyl, and the like, or multiple ring structures such as adamantanyl, and the like. The term "substituted cycloalkyl" refers to cycloalkyl groups having from 1 to 5 substituents, or from 1 to 3 substituents, selected from alkyl, substituted alkyl, alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, eterocyclooxy, hydroxyamino, alkoxyamino, nitro, -SO-alkyl.

"Heterocyclyl" refer to a saturated or unsaturated group having a single ring or multiple condensed rings, including fused bridged and spiro ring systems, and having from 3 to 20 ring atoms, including 1 to 10 hetero atoms. These ring atoms are selected from the group consisting of nitrogen, sulfur, or oxygen, wherein, in fused ring systems, one or more of the rings can be cycloalkyl, aryl, or heteroaryl, provided that the point of attachment is through the non-aromatic ring. In certain embodiments, the nitrogen and/or sulfur atom(s) of the heterocyclic group are optionally oxidized to provide for the N-oxide, -S(O)-, or -SO2- moieties. Examples of heterocycles and heteroaryls include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, tetrahydrofuranyl, and the like. Unless otherwise constrained by the definition for the heterocyclic substituent, such heterocyclic groups can be optionally substituted with 1 to 5, or from 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-substituted alkyl, -SO-aryl, -SO-heteroaryl, -SO2-alkyl,-SO2-substituted alkyl, -S02-aryl, -SO2-heteroaryl, and fused heterocycle.

The substitution of the mono-, or di-substituted 1,2,4,5-tetrazines for practicing the method of the present invention is highly variable and not limited to any specific chemistry or substitution pattern. If a substituent can be covalently attached to the C3 and/or C6 position of 1,2,4,5-tetrazines it is useful for the present invention. In embodiments the mono-, or di-substituted 1,2,4,5-tetrazines are selected from the group consisting of tetrazines, wherein the one or two substituents in positions 3 and 6 are independently selected from the substituents listed herein.

In embodiments, the method of the present invention is one, wherein the compound is a (poly)peptide comprising at least one α-keto-β³-glycine(aminopyruvate) that is located within a cell expressing an rSAM enzyme, and the tetrazine compound is added extracellularly, wherein optionally the (poly)peptide and/or the rSAM enzyme are secreted from the cell.

In embodiments, when the α-keto-β³-glycine(aminopyruvate) comprising compound for practicing the method of the invention is a (poly)peptide, it is optionally located within a cell expressing an rSAM enzyme for its production, and the 1,2,4,5-tetrazines are added extracellularly, and optionally the (poly)peptide and/or the rSAM enzyme are secreted from the cell. Cells for producing α-keto-β³-glycine(aminopyruvate) comprising compounds for practicing the method of the invention may vary widely as long as they express the (poly)peptide with the required tripeptide motif for rSAM modification and the desired rSAM.

The rSAM enzymes and their target polypeptides for α-keto-β³-glycine(aminopyruvate) introduction may be expressed in suitable prokaryotes and eukaryotes by conventional and well-known methods. Examples of suitable host cells include prokaryotic and eukaryotic unicellular organism, such as bacteria, optionally *E. coli* strains, *Bacillus spp.,* optionally *B. subtilis,* yeast and fungi, optionally S. *cerevisiae, Pichia spp.* And also host cells originally derived from higher organisms such as insects, vertebrates, including mammals, optionally CHO, HEK cells, may be used as the host cells for target polypeptide and rSAM enzyme expression and optionally secretion. Suitable mammalian cell lines include, but are not limited to, HeLa cells (e.g., American Type Culture Collection (ATCC) No. CCL-2), CHO cells (e.g., ATCC Nos. CRL9618 and CRL9096), CHO DG44 cells (Urlaub (1983) Cell 33:405), CHO-K1 cells (ATCC CCL-61), 293 cells (e.g., ATCC No. CRL-1573), Vero cells, NIH 3T3 cells (e.g., ATCC No. CRL1658), Huh-7 cells, BHK cells (e.g., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells. Suitable expression systems include bacterial, yeast, insect cell and mammalian cell derived expression systems.

The expressed target polypeptides and rSAM enzymes can be recovered by any appropriate means known in the art. For example, protein purification procedures may be employed, e.g. as described in the Guide to Protein Purification, (Deuthser ed.) (Academic Press, 1990). Optionally, a lysate may be prepared from a cell comprising the expression vector expressing the desired polypeptide, and purified using high performance liquid chromatography (HPLC), exclusion chromatography, gel electrophoresis, affinity chromatography.

Non-limiting examples of recombinant cells suitable for expressing an extrinsic rSAM enzyme are selected from *E. coli,* optionally *E. coli* BL21, *E. coli* Rosetta, *E. coli* Tuner*, Pleurocapsa sp.* PCC 7319, proteobacteria, *Frankia* spp., actinomycetes, *Thiothrix* spp., *Pleurocapsa* sp. PCC 7327, and mammalian cell lines, optionally CHO, HeLa, and HEK cells.

In embodiments the method of the present invention is one, wherein
(1) the compound comprising at least one α-keto-β³-amino acid (aminopyruvate) is a (poly)peptide;
(2) the 1,2,4,5-tetrazine is a mono- or disubstituted 1,2,4,5 tetrazine;
(3) the carbon substituents corresponding to carbon positions 3 and 6 on 1,2,4,5-tetrazine are selected from the substituents cited herein, optionally analytical, purification, diagnostic, medicinal, reactive, solubilization, and stabilization substituents;
(4) the rSAM enzyme is selected from the enzymes listed herein, optionally an rSAM enzyme having an amino acid sequence of SEQ ID NO: 2 or 4; and/or
(5) the rSAM enzyme expressing cell is selected from the cells listed herein, optionally the cell is selected from the group of cells consisting of *E. coli,* optionally *E. coli* BL21, *E. coli* Rosetta, *E. coli* Tuner, *Pleurocapsa sp.* PCC 7319, proteobacteria, *Frankia* spp., actinomycetes, *Thiothrix* spp., *Pleurocapsa* sp. PCC 7327, yeast cells, optionally S. cerevisiae, and mammalian cell lines, optionally CHO, HeLa, and HEK cells.

In a second aspect the invention relates to a (poly)peptide, (poly)peptide derivative or peptidomimetic comprising at least one pyridazine, the pyridazine optionally forming part of the (poly)peptide, (poly)peptide derivative or peptidomimetic backbone, optionally obtainable or obtained by a method of the invention as described herein.

In embodiments the (poly)peptide, (poly)peptide derivative or peptidomimetic of the invention comprises one or two analytical, purification, diagnostic, medicinal, reactive, solubilization and/or stabilization substituents bound to at least one pyridazine, optionally one or two substituents as mentioned herein.

In a further aspect the invention pertains to the use of a pyridazine comprising (poly)peptide, (poly)peptide derivative or peptidomimetic as mentioned herein, optionally having the pyridazine in the backbone of the molecule, for compound identification, quantification, purifycation, stabilization and/or solubilization.

In further embodiments of this aspect the invention pertains to the use of a pyridazine comprising (poly)peptide, (poly)peptide derivative or peptidomimetic as mentioned herein, optionally having the pyridazine in the backbone of the molecule, for use in therapy or diagnosis.

Moreover, the present invention also reads on a method of treatment or diagnosis comprising the step of administering a (poly)peptide, (poly)peptide derivative or peptidomimetic as mentioned herein, in a physiologically effective amount to a patient in need thereof.

Last but not least, it is emphasized that the method of the present invention for the first time allows for introducing non-, mono- and disubstituted 1,2,4,5 tetrazines into the backbone of (poly)peptides, (poly)peptide derivatives or peptidomimetics under controlled, mild and high yield conditions.

The term "(poly)peptide backbone", as used herein, indicates the longest continuous chain of covalently bonded amino acids including modified amino acid(s) in the chain. Of course, this understanding applies in analogy to (poly)peptide derivatives and peptidomimetics.

In the following the invention will be illustrated by way of examples and figures, none of which are to be interpreted as limiting the invention beyond the scope of the appended claims.

### Figures

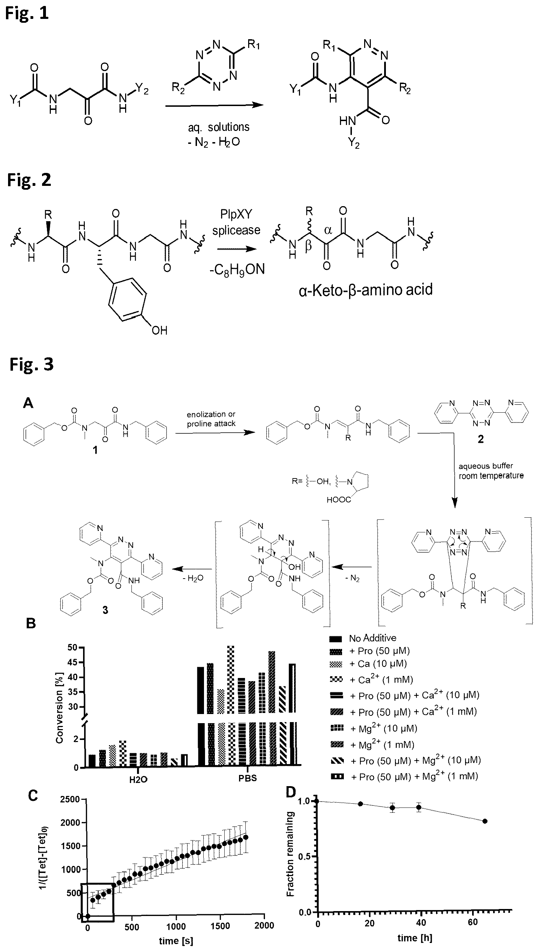

**Fig. 1** is a simple reaction scheme illustrating the reaction of aminopyruvate-comprising compounds, e.g. a (poly)peptide, with mono-, di-, -substituted 1,2,4,5-tetrazine derivatives to yield corresponding pyridazine conjugates, in a so-called TyrEx cycloaddition, which was demonstrated in aqueous and organic solutions and also in living organisms, e.g. bacteria. Substituents R1 and R2 may be functional, e.g. labels, proteomic tags, drugs, etc. Y1 or Y2 can be (parts of) a (poly)peptide.
**Fig. 2****.** is a reaction scheme illustrating the (poly)peptide splicing by a PlpXY splicease enzyme (rSAM) for posttranslational excision of a tyramine equivalent (C₈H₉ON) from the tyrosine-glycine motif, wherein R is the side chain of an α-keto-β³-amino acid (amino pyruvate).
**Fig. 3** A) illustrates the possible reaction mechanism between model compound **1** and 3,6-di-2-pyridyl-1,2,4,5-tetrazine **(2)** to yield pyridazine **3.** B) is a graph showing the effect of medium, proline, and divalent metal ion additives on the pyridazine **3** yield, estimated by MS product ion abundance and calculated apparent conversion. PBS is phosphate-buffered saline pH 7.4. C) is a graph showing the reaction kinetics measured in PBS with calcium additive by visible light spectrometry. The decrease in absorbance at 525 nm of 3,6-di-2-pyridyl-1,2,4,5-tetrazine was measured over time, adjusted to the degradation of tetrazine, and fitted to a second-order reaction kinetic equation. A burst phase is highlighted by the rectangle. D) Stability of conjugate **3** over time upon reaction completion, assessed by relative MS intensity. Error bars represent standard deviations.

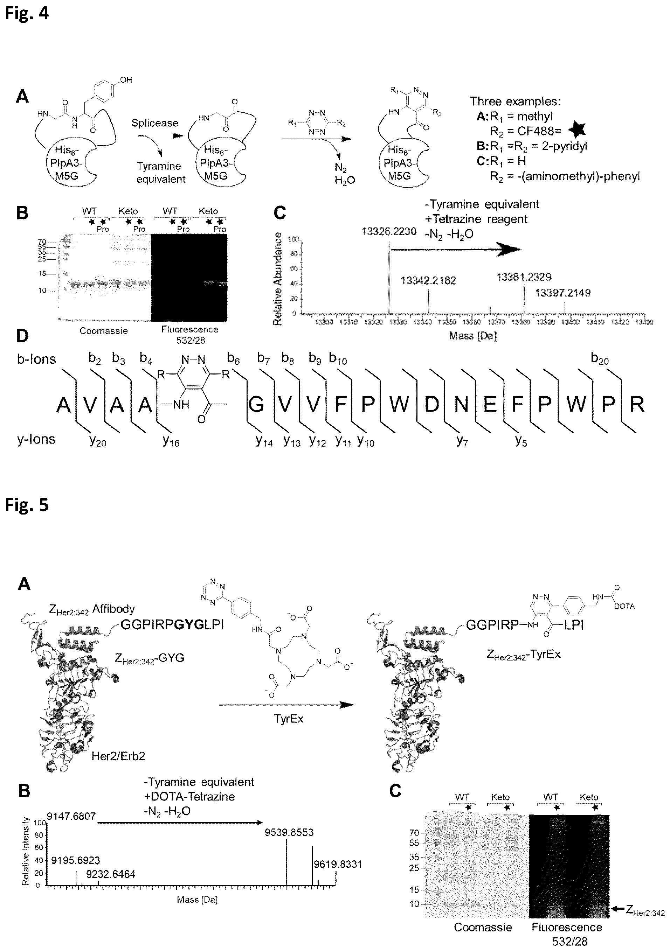

**Fig. 4.** A) is a scheme illustrating the overall tyramine excision (TyrEx) strategy for *in vivo* protein labelling: a splicease expressed in the cell site-specifically installs aminopyruvate in the target protein at a GYG motif. The post-translationally modified protein reacts with a tetrazine derivative to form the pyridazyl protein conjugate. Tyramine equivalent mass loss in red = -135.0757 Da (-C₈H₉NO). B) is a photo demonstrating the conjugation of His₆-PIpA3-M5G with a fluorescent tetrazine probe analysed by SDS-PAGE. WT: unmodified HiS₆-PlpA3-M5G; Keto: HiS₆-PlpA3-M5G co-produced with PlpXY to yield aminopyruvate. The protein gel fluorescence was imaged using 488 nm excitation and 532/28 nm emission filter. C) is a graph for deconvoluted ESI-MS showing ions for the substrate (His₆-PIpA3-M5G, m/z calcd. 13326.2112) and the cycloaddition product using tetrazine **2** (*m*/*z* calcd. 13381.2071). Tetrazine **2** in blue mass (m/z 236.0810). D) is graph showing the observed y- and b-ions for the ESI-MS-MS fragmentation of a trypsinized reaction product localizing the cycloaddition product.
**Fig. 5****.** Illustrates the production of a Her2-binding Affibody radiolabelling conjugate by TyrEx cycloaddition. A) is a cartoon visualization of the Z_{Her2:342} Affibody bound to Her2/ErbB2 based on crystal structures³⁸ (PDB accession 3MZW) with the C-terminal splicease tag. *In vivo* splicing and subsequent *in vitro* conjugation to a DOTA-tetrazine derivative yields conjugates for radiolabelling. B) shows the deconvoluted MS spectrum confirming the formation of the DOTA conjugate (HiS₆-Z_{Her2:342}-TyrEx-DOTA, *m*/*z* calcd. 9539.8277). Tyramine equivalent mass loss in red = -135.0757 Da (-C₈H₉NO). DOTA-Tetrazine mass 573.2659 Da. C) is a picture of an SDS-PAGE analysis of the spliced Z_{Her2:342} Affibody labelled with CF488 tetrazine. The left gel shows Coomassie stained protein bands, the right gel shows gel fluorescence using 488 nm excitation and 532/28 nm emission filter. The keto lane was loaded with Affibody co-produced with PlpXY, in WT lane Affibody produced without PlpXY. The star symbol denoted the addition of CF488 tetrazine. Two bands per lane arise from nonspecific binding and dye impurities.

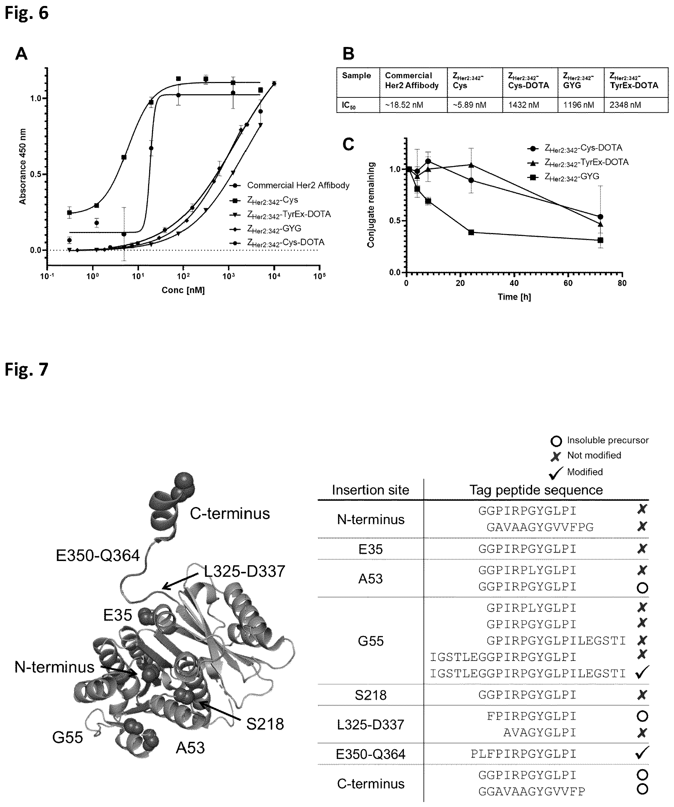

**Fig. 6** shows the biochemical characterization of Z_{Her2:342}-TyrEx-DOTA. A) is graph relating to an enzyme-linked immunosorbent assay (ELISA) for Her2 immobilized on a 96-well plate. B) shows ELISA-derived IC₅₀ values against Her2 for Z_{Her2:342}-TyrEx-DOTA, its precursor Z_{Her2:342}-GYG, Z_{Her2:342}-Cys-DOTA and its precursor Z_{Her2:342}-Cys. C) is a graph showing stability measurements of Z_{Her2:342}-TyrEx-DOTA, its precursor protein, and Z_{Her2:342}-Cys-DOTA in human blood plasma over time at 37 °C, judged by LC-MS.
**Fig 7****.** Shows the modelled FtsZ protein structure with studied tag locations. **A)** is a ribbon diagram of the X-ray structure of *Escherichia coli* FtsZ, N-terminal globular domain (PDB accession 6UMK). The tag insertion sites (*E. coli* numbering) are shown. A 50-amino acid segment extending over E350 region is shown as an unstructured flexible peptide. The model was constructed using PyMol.⁴² **B)** is a table listing Tag sequences tested for splicease activity.
The cross symbol in red denotes the not accepted motif sequence, the green check-mark denotes the spliced motif.

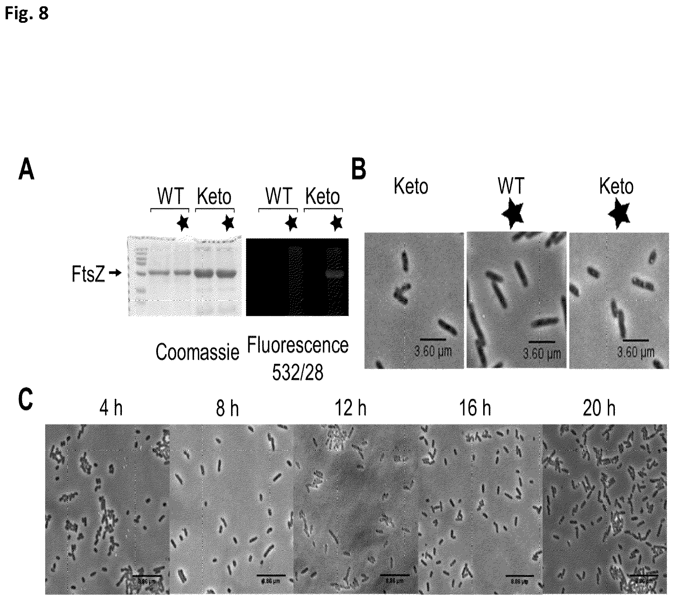

**Fig. 8****.** Shows the imaging of FtsZ fluorescently labelled by TyrEx cycloaddition. Samples labelled Keto were co-expressed with the splicease PlpXY. The star symbol denoted the addition of CF488 tetrazine. A) is an SDS-PAGE analysis of CF488 labelled FtsZ G55. The left gel shows Coomassie stained protein bands, the right gel shows gel fluorescence using 488 nm excitation and 532/28 nm emission filter. B) are *in situ* fluorescence micrographs of *E. coli* cells harbouring fluorescent FtsZ G55. C) are fluorescence micrographs of cells cultured to the indicated time points. Overlaid images of brightfield illumination and 488 nm are shown.

### Examples

### Plasmid construction

Protein expression plasmids were constructed following Gibson Assembly^{®} Cloning (for genes >100 base pairs), or Q5^{®} site-directed mutagenesis (for genes <100 base pairs) protocol provided by New England Biolabs (USA). Gene assembly fragments were designed by NEBuilder tool online. Overlapping mutagenic primers were designed by NEBaseChanger tool online. Plasmids were transformed into chemically competent *E. coli* DH5α strain (Invitrogen) and grown on selective LB agar, then inoculated in liquid LB media. Plasmids were isolated from fresh overnight cultures and gene-of-interest was sequenced-verified. All plasmids are under IPTG regulation. pACYCDuet encodes chloramphenicol resistance, pRSFDuet kanamycin resistance, and pRM006 ampicillin resistance.

**Table 1. Plasmids used for the examples**

| Plasmid | Description |
|---|---|
| *p*/*pA2*/pACYCDuet-1 | Encodes the native PlpA2 precursor protein with an N-terminal His6-tag (from here on referred to as His6-PlpA2 (Morinaka, 2018) |
| *p*/*pA3*/pACYCDuet-1 | Encodes the native PlpA3 precursor protein with an N-terminal His6-tag (from here on referred to as His6-PlpA3 (Morinaka, 2018) |
| *plpXY*/*pRSFDuet* | Encodes the Splicease PlpX together with its accessory protein PlpY under IPTG regulation and with kanamycin resistance (referred to as PlpXY) (Morinaka, 2018) |
| *plpA2* M5G/pACYCDuet-1 | PlpA2 protein with a point mutation at position 5 of the core peptide to glycine with an N-terminal His6-tag (referred to as His6-PlpA2-L5G) |
| *plpA3* M5G/pACYCDuet-1 | PlpA3 protein with a point mutation at position 5 of the core peptide to glycine with an N-terminal His6-tag (referred to as His6-PlpA2-M5G) |
| *FtsZ*/*pRM006* | FtsZ protein expression plasmid was a gift from Jie Xiao (Addgene plasmid # 98922) |
| *FtsZ G55*/*pRM006* | FtsZ gene harbouring splicing motif insertion between G55-Q56 |
| *FtsZ E350*/*pACYCDuet-1* | FtsZ gene harbouring splicing motif substitution between E350-Q364 |
| His₆-ZHer2:342-GYG /*pACYCDuet-1* | His₆-tagged Z_{Her2:342} Affibody gene with C-terminal splicing motif |

### Example 1- Synthesis of benzyl (2-hydroxyethyl)(methyl)carbamate

To a solution of 2-(methylamino)ethan-1-ol (1.0 mL, 13 mmol, 1 eq.) and triethylamine (2.5 mL, 18 mmol, 1.4 eq.) in anhydrous DCM (4 mL) at 0 °C was added benzyl carbonochloridate (2.0 mL, 14 mmol, 1.05 eq.) over 30 minutes under an inert atmosphere. The reaction mixture was allowed to warm up to room temperature and stirred for 2 hours. Additional DCM (4 mL) was added and the organic phase was washed with 1 M HCl (8 mL). The aqueous phase was back-extracted with DCM (8 mL). The combined organic phases were washed with H₂O (8 mL) and brine (8 mL), dried over magnesium sulfate, filtered, and evaporated under reduced pressure to provide benzyl (2-hydroxyethyl)(methyl)carbamate as a brown oil (2.085 mg, 77%).

### Example 2 - Synthesis of benzyl methyl(2-oxoethyl)carbamate

After stirring a solution of benzyl (2-hydroxyethyl)(methyl)carbamate (500 mg, 2.4 mmol, 1 eq.), DMSO (1.7 mL, 24.0 mmol, 10 eq.), and DIPEA (2.0 mL, 11.5 mmol, 5 eq.) at 0 °C for 10 minutes under an inert atmosphere, sulfur trioxide pyridine complex (1.9 g, 11.5 mmol, 5 eq.) was added and the mixture was stirred for 1.5 hours. The reaction was quenched with 1 M HCl (5 mL) and the organic phase extracted and washed with sat. aq. NaHCO₃ (5 mL) and brine (5 mL). The solvent was evaporated under reduced pressure and benzyl methyl(2-oxoethyl)carbamate was obtained as a yellow oil (310 mg, 60%).

### Example 3 - Synthesis of benzyl (4-(benzylamino)-3,4-dioxobuty)(methyl)carbamate (1)

To a solution of *N*-methylhydroxylamine hydrochloride (74 mg, 0.88 mmol, 1.1 eq.), NaHCO₃ (134 mg, 1.57 mmol, 2 eq.), and 4 Å molecular sieves (600 mg) in 0.8 mL anhydrous MeOH was added benzyl methyl(2-oxoethyl)carbamate (160 mg, 0.8 mmol, 1 eq.) and the mixture was stirred for 30 minutes at room temperature. To this solution was added benzyl isocyanide (100 mg, 0.84 mmol, 1.05 eq.) and AcOH (400 µL, 7.20 mmol, 9 eq.), and the mixture was stirred for 24 hours at room temperature. The reaction mixture was filtered, and the solvent evaporated under reduced pressure. The crude product was purified by silica chromatography (12 g, 60% EtOAc in n-Hexan, R_{f}=0.6) to obtain 1 as a yellow oil (50 mg, 20%). ¹H-NMR (400 MHz, CDCl₃): δ = 7.40 - 7.27 (m, 10H), 5.12 (2 s, 2H), 4.73 - 4.68 (2 s, 2H), 4.47 (dd, J = 11.2, 6.1 Hz, 2H), 2.97 (d, J = 1.1 Hz, 3H) ppm (2 Rotamers). ¹³C-NMR (101 MHz, CDCI3) δ = 171.23, 128.89, 128.50, 128.45, 128.05, 127.95, 127.84, 127.80, 77.33, 77.01, 76.69, 67.58, 67.45, 60.42, 55.39, 54.89, 43.33, 35.99, 35.47, 21.05, 20.68, 14.19.

### Example 4 - Synthesis of 2,2',2"-(10-(2-((4-(1,2,4,5-tetrazin-3-yl)benzyl)amino)-2-oxoethyl)-1,4,7,10-tetraazacyclododecane-1,4,7-triyl)triacetate(DOTA-Tetrazine)

1,4,7,10-Tetraazacyclododecane-1,4,7,10-tetraacetic acid mono-N-hydroxysuccinimide ester (17.1 mg, 22.5 µmol, 1 eq.) and (4-(1,2,4,5-Tetrazin-3-yl)phenyl)methanamine hydrochloride (5 mg, 22.5 µmol, 1 eq.) were dissolved in 0.75 mL DMF in a pear-shaped flaks and added with DIPEA (14.54 mg, 112.5 µmol, 5 eq.) and stirred 16 hours at room temperature. The solvent was evaporated under reduced pressure and the reaction mixture dissolved in 50% acetonitrile/H₂O. DOTA-Tetrazine was purified by reverse-phase HPLC on a Phenomenex Luna 5u C18 column (250 × 10 mm) and the fractions containing the product dried to obtain a pink solid (4.9 mg, 40%). ¹H-NMR (400 MHz, DMSO-d₆): δ = 10.62 (s, 1H), 9.07 (s, 1H) 8.50 (d, J = 8 Hz, 2H), 7.61 (d, J = 8 Hz, 2H), 4.50 (d, J = 5.6 Hz, 2H), 4.04 (d, J = 15.8 Hz, 4H), 3.64 (b, 4H), 3.43 - 3.06 (m, 16H) ppm.

### Example 5 - Small Molecule Studies with pyridazine compound 3

For additive studies, model compound 1 (100 µM) was dissolved H₂O or PBS and added with 3,6-di-2-pyridyl-1,2,4,5-tetrazine (100 µM), then supplied with additives proline (50 µM), CaCl₂ (10 µM or 1 mM) and/or MgCl₂ (10 µM or 1 mM) to prepare pyridazine compound 3. The samples were incubated overnight at 37 °C and analysed by LC/MS.

### Example 6 - Stability measurements

To measure the stability of pyridazine compound 3 in aqueous solutions, model compound 1 (100 µM) and 3,6-di-2-pyridyl-1,2,4,5-tetrazine (100 µM) were dissolved in 200 µL H₂O and incubated at 37 °C for three days. The sample was then analysed repeatedly by LC/MS with method B over the course of 3 days. A sample was measured approximately every 12 hours. From the MS data, the intensity of the signal at m/z = 531.2139 at a retention time of 2.0 to 3.5 minutes was extracted and plotted versus time. Measurements were done in triplicates.

### Example 7 - Kinetics measurements

For kinetic measurements, 196 µL trypsin buffer was added with 2 µL of a 10 mM stock solution of compound 1 in DMSO (final concentration 100 µM) and 2 µL of a 10 mM stock solution of 3,6-di-2-pyridyl-1,2,4,5-tetrazine in DMSO (final concentration 100 µM) in a quartz cuvette. The sample was mixed well and absorption at 525 nm was monitored over time, measuring every minute. Measurements were done in triplicates. In a control sample, decrease in absorption over time of 3,6-di-2-pyridyl-1,2,4,5-tetrazine alone was measured and fitted to a first-order reaction equation, yielding a rate constant of 2.96×10⁻⁵ s⁻¹. This was subtracted from the kinetic measurements. The obtained absorbance values were converted to concentrations through a standard curve and were plotted as the reciprocal concentration of the tetrazine reagent versus time to yield a second-order rate constant. Data analysis and statistical analysis were done in Microsoft Excel (2016) and Prism 7 (GraphPad).

### Example 8 - Protein expression and purification

A falcon tube containing 5 mL of LB medium was inoculated with *E. coli* BL21 (DE3) harboring appropriate plasmids cells taken from previously prepared glycerol stocks or from single colonies on agar plates and supplemented with the appropriate antibiotics. The culture was shaken at 180 to 220 rpm overnight at 37 °C.

On the next day, 30 to 800 mL TB medium containing the appropriate antibiotics were inoculated with 1% v/v of this overnight culture and shaken at 37 °C until an OD₆₀₀ of 1.6 to 1.8 was reached. After cooling the cultures at 4 °C for at least 20 minutes, 1 mM of IPTG was added and the cultures were incubated on a shaker at 180 to 220 rpm at 16 to 24 °C for approximately 16 to 20 hours. Subsequently, the cultures were centrifuged at 5000 g for 10 minutes at 4 °C. The supernatant was discarded, and the cell pellets resuspended in 1 to 25 mL NPI-10 buffer.

All volumes were adjusted to scale with the culture volume (e.g. 1 mL NPI-10 buffer for resuspension was used for 30 mL cultures, 10 mL NPI-10 buffer were used for resuspension of cells grown in 300 mL medium).

Cells resuspended in NPI-10 buffer were sonicated for 10 times 10 seconds, with 10 seconds of pause in between, at an amplitude of 25 to 40. A sonication tip with a radius of 2 to 6 mm was used, depending on the volume to be sonicated. The resulting suspensions were kept on ice and centrifuged at 21000 g for 30 minutes at 4 °C.

The supernatant was transferred to a new tube and 125 µL to 2 mL of Protino^{®} Ni-NTA Agarose (Macherey-Nagel) was added. The samples were slowly shaken on a rotor at 10 rpm for at least 30 minutes. An appropriate column was prewashed with NPI-10 buffer, the sample added, washed twice with NPI-10 (500 µL to 10 mL), twice with NPI-20 (500 µL to 10 mL), and the protein eluted with NPI-250 (550 µL to 10 mL) and collected. Elution fractions were digested with appropriate endoproteinases. Protein splicing was analyzed by high-resolution LC-MS. Samples were stored at -20 °C for further use.

### Example 9 - General protein labelling reactions

Affinity-purified proteins containing aminopyruvate sites were generally incubated in NPI-250 buffer with 100 µM (DOTA-Tetrazine, 3,6-di-2-pyridiyl-1,2,4,5-tetrazine) or 10 µM (CF488-Tetrazine) for 18 hours at 37 °C. Reactions were then directly analysed by LC-MS for full proteins or first digested with appropriate endoproteases.

Proteins labelled with a tetrazine probe conjugated to a fluorescent dye (CF^{®}488A tetrazine, Chemie Brunschwig AG) were run on SDS page after incubation overnight at 37 °C. The gels were analysed by a fluorescent imager with a band pass filter 532/28 (ChemiDoc MP, BioRad), followed by staining with Coomassie Brilliant Blue and subsequent destaining (10% AcOH, 30% MeOH, 60% H₂O) overnight.

### Example 10 - Z_{Her2:342}-TyrEx-DOTA and Z_{Her2:342}-Cys-DOTA conjugation and purification

Z_{Her2:342}-TyrEx-DOTA conjugation reaction was prepared by adding DOTA-Tetrazine (10 µL, 1 mM solution in DMSO) to the TyrEx-tagged protein solutions in NPI-250 buffer. Following incubation overnight at 37 °C reaction mixtures were loaded to solid phase extraction columns (Strata-X 100mg/3mL, Phenomenex) pre-equilibrated first 10 column volumes with acetonitrile and subsequently 10 column volumes H₂O. Columns were washed with 10 column volumes 5% acetonitrile/H₂O + 0.1% formic acid. Protein conjugates were eluted with 10 column volumes 30% acetonitrile/H₂O + 0.1% formic acid. Resulting elutions were purified by reversed-phase HPLC on a Phenomenex Luna 5u Phenyl-Hexyl column (250 × 4.6 mm) with solvent A: H2O + 0.1% TFA, solvent B: acetonitrile + 0.1% TFA by the following gradient method: B 5% ramped up to 25% over the first 2 minutes, then slow gradient to 29% at 14 minutes, then to 100% B over 1 minute, 100% B for 2 minutes, then again 5% B for 3 more minutes to equilibrate. Fractions were analysed by LC-MS for purity and pure fractions combined.

Z_{Her2:342}-Cys-DOTA was prepared from Ni-NTA elutions (2 mg/mL) by first reducing the protein solutions with 30 µM DTT for 2 h at 37 °C and removal of excess DTT by PD-10 desalting columns pre-equilibrated with 20 mM ascorbic acid. 900 µL of the resulting elution were mixed with DOTA maleimide (200 µL ,1 mg/mL) and NH₄OAc (300 µL, 1 M, pH 5.5). The reaction mixtures were incubated overnight at 37 °C, desalted by PD-10 desalting columns preequilibrated with 200 mM NH₄OAc, then purified by reversed-phase HPLC with the same conditions as Z_{Her2:342}-TyrEx-DOTA.

Purified proteins were dried on a small-scale rotary evaporator (Eppendorf Concentrator 5301) and resuspended in PBS. Protein concentrations were measured by ROTI^{®} Nanoquant assay (Carl Roth).

### Example 11- ELISA

Z_{Her2:342}-TyrEx-DOTA (5 µM), ZHer2:342-GYG (1.875 µM), Z_{Her2:342}-Cys (5 µM), Z_{Her2:342}-TyrEx-Cys-DOTA (10 µM), Her2 commercial Affibody (5 µM) were prepared as solutions in PBS at the given concentrations. The assay was carried out with a Her2-coated plate and buffers from a *EDI*^{Tm} Humanized Anti-Her2/neu (Herceptin/trastuzumab) ELISA Kit (Epitope Diganostics). A 4x dilution series of each sample in 200 µL was prepared in a 96-well transfer plate with the supplied assay buffer. Samples (100 µL) were then transferred to the Her2-coated plate and incubated at 22 °C on an orbital shaker at 400 rpm for 90 minutes. Each well was washed 5 times with the supplied wash buffer and 100 µL Goat Anti-Affibody IgG (Affibody SE) 0.5 µg/mL in assay buffer was added to each well, then incubated at 22 °C at 400 rpm for 60 minutes. Each well was washed 5 times with the supplied wash buffer and 100 µL Anti-goat IgG (Fc specific)-Peroxidase antibody (Sigma Aldrich) 50 ng/mL was added to each well, then incubated at 22 °C at 400 rpm for 60 minutes. Each well was washed 5 times with wash buffer and 100 µL of the supplied HRP substrate solution was added to each well, the plate wrapped with aluminium foil and incubated at room temperature in the dark for 30 minutes. The reaction was stopped by the addition of 100 µL of the supplied stop solution. Absorption at 450 nm was measured with a Victor3 (PerkinElmer Inc.) spectrophotometer. Binding curves and sigmoidal fits were constructed by Graphpad Prism 8.2.

### Example 12 - Blood plasma stability

Z_{Her2:342}-TyrEx-DOTA, Z_{Her2:342}-GYG, and Z_{Her2:342}-Cys-DOTA (135 µg/mL, 25 µL) and 25 µL human blood plasma (Sigma Aldrich) were mixed and incubated at 37 °C in triplicates. After 0, 1, 4, 8, 24, and 72 hours 10 µL were removed from the solutions and stored at -80 °C until LC-MS analysis. Samples were mixed with 25 µL of 75% acetonitrile in water to precipitate blood plasma proteins. Samples were subjected to LC-MS after centrifugation (2x 20 000 g for 10 min). Intensities of deconvoluted mass ions for Z_{Her2:342}-TyrEx-DOTA, Z_{Her2:342}-GYG and Z_{Her2:342}-Cys-DOTA respective conjugates were recorded. Intensities were normalized to the t = 1 hour time point and plotted against incubation time.

### Example 13 - FtsZ-splicease motif fusion cloning

Protein expression plasmid encoding His₆-SUMO tagged FtsZ was purchased from Addgene (#98922). Splicing motif gene was encoded in five FtsZ sites between amino acid residues E35-G35, A53-V54, G55-Q56 and SUMO-FtsZ junction.

The FtsZ gene was subcloned into pACYCDuet-1 vector, multiple cloning site 1 in frame with N-teminal His₆-tag by Gibson assembly. The linear FtsZ gene was PCR amplified from the pRM006 vector with overlapping primers to the target vector. Primers were designed in NEBuilder tool online. Assembly was done following protocol by New England Biolabs. Splicing motif gene was inserted between L325-D337, E350-Q364 and at N- and C-terminus of FtsZ by full plasmid amplification with mutagenic primers.

### Example 14- Labeling proteins with a fluorescent tetrazine in live E. coli and preparation for microscopy

*E. coli* BL21 (DE3) cells harboring precursor and splicease plasmids were grown according to the protein expression procedure. Cells were taken from the culture and diluted to an OD₆₀₀ of 0.4 - 0.6 with PBS (pH 7.4, final volume: 100 µL). The samples were centrifuged (7000 g, 5 min), the supernatant discarded, and the cells resuspended in PBS (100 µL). Samples were incubated with 0.3% TritonX-100 at room temperature for 30 minutes, then washed twice with PBS. Samples were incubated with 100 µM CF^{®}488A tetrazine for 60 minutes at 16 °C, then centrifuged (7000 g, 5 min), the supernatant discarded, and the cells resuspended in PBS (100 µL). This wash was repeated twice. Samples (1-3 µL) were then added to a cover slip and covered with 1.5% w/v agarose pad for imaging. From these samples, regions of interest were located using the bright-field mode on the microscope and z-stacks were recorded in bright-field and at 488 nm (200 mW, 30% power, 400 ms).

Samples were imaged on an Axiovert 200m (inverse) microscope, equipped with a Yoko-gawa CSU-X1 spinning-disk confocal unit and a LUDL BioPrecision2 stage with Piezo Focus. Images were acquired with a sCMOS camera (Orca Flash 4.0 V). Diode-pumped solid-state lasers (DPSS) were used as light sources, where the 488 nm (200 mW) laser was used. A 100× 1.4 CFI Plan Apo Oil objective was used for all image acquisitions. Emitted light was filtered using the GFP (ET 525/50) filter. The microscope was operated using the VisiVIEW (Metamorph) software. Image analysis was performed with Fiji (ImageJ).

### Example 15 - High-resolution mass spectrometry

Mass spectra were acquired on an LTQ Orbitrap XL or Qexactive (Thermo Fisher Scientific, USA) spectrometer by using heated electrospray ionization (HESI). The following methods were used for analysis on LC/MS:
Method A: solvent A, H₂O + 0.1% formic acid; solvent B, MeCN + 0.1% formic acid; column, Phenomenex Kinetex 2.6 µm C18-XB 100 Å (150 × 4.6 mm); flow rate, 1.0 mL/min; gradient: 95:5 A/B for 0.5 min ramped to 45:55 A/B over 20 min).
Method B: solvent A, H₂O + 0.1% formic acid; solvent B, MeCN + 0.1% formic acid; column, Phenomenex Kinetex 2.6 µm C18-XB 100 Å (150 × 4.6 mm); flow rate, 1.0 mL/min; gradient: 10:90 A/B for 1 min ramped to 5:95 A/B over 7 min).
Method C: solvent A, H₂O + 0.5% formic acid; solvent B, 1-Propanol + 0.5% formic acid; column, Aeris Widepore 3.6 µm C4 (50 × 2.1 mm); flow rate, 0.7 mL/min; gradient: 95:5 A/B for 2 min ramped to 50:50 A/B over 16 min).

## Claims

1. A method for preparing pyridazine compounds, optionally pyridazine comprising (poly)peptides, (poly)peptide derivatives and peptidomimetics comprising the step of reacting
(i) a compound, optionally a (poly)peptide, (poly)peptide derivative or peptidomimetic comprising at least one α-keto-β³-glycine(aminopyruvate) with
(ii) a non-, mono-, or di-substituted 1,2,4,5-tetrazine
under conditions suitable for the formation of at least one pyridazine conjugate.

2. The method of claim 1, wherein the conditions are selected from the group consisting of aqueous compositions, pure water, organic compositions, alcoholic compositions, optionally pure methanol, aqueous buffered compositions, physiological compositions, phosphate-buffered saline (PBS), NPI-250 (50 mM sodium phosphate, 300 mM NaCl, 250 mM imidazole, pH 8), MES (200 mM MES, pH 5.5), *in vitro* and *in vivo* cellular conditions, *E. coli* lysate, *E. coli* cytosol, ambient temperatures, temperatures of 2 to 40, 5 to 37 °C, 15 to 37°, and a pH of 2 to 11, 4 to 10, 5 to 8.5.

3. The method of claim 1 or 2, further comprising the step of preparing the compound (i), wherein the compound is a (poly)peptide, polypeptide derivative or peptidomimetic comprising at least one α-keto-β³-glycine(aminopyruvate), by reacting
(a) (poly)peptides comprising one or more of amino acid motifs GYG, SYG, GYA, SYA, GFG, SFG, GFA, SFA, optionally one of amino acid motifs PIRPXYGLPI or AVAAXYGVVFP, wherein X is glycine or serine, with
(b) a radical S-adenosyl methionine (rSAM) enzyme that excises tyramine from at least one of the amino acid motifs in (a) to produce an α-keto-β³-glycine(aminopyruvate),
wherein the (poly)peptides and/or the rSAM enzyme are produced by cells expressing and optionally secreting the (poly)peptides and/or the rSAM enzyme, and
wherein the α-keto-β³-glycine(aminopyruvate) is produced within or outside the cells.

4. The method of claim 3, wherein the rSAM enzyme is selected from the group consisting of
(a) rSAM enzymes comprising, optionally identical to, one of amino acid sequences of SEQ ID NOs. 2 or 4;
(b) rSAM enzymes having an amino acid sequence identity of at least 70 or 80 %, optionally at least 90 or 95 %, with one of amino acid sequences of SEQ ID NOs. 2 and 4;
(c) rSAM enzymes that are functional fragments and/or functional derivatives of (a) or (b);
(d) rSAM enzymes encoded by a nucleic acid sequence of SEQ ID NOs: 1 or 3;
(e) rSAM enzymes encoded by a nucleic acid sequence of at least 80 or 90 % sequence identity, optionally at least 95 % or 98 % sequence identity, with one of SEQ ID NOs: 1 or 3, optionally over the whole sequence;
(e) rSAM enzymes encoded by a nucleic acid sequence that hybridizes to one of SEQ ID NOs: 1 or 3 under stringent conditions;
(f) rSAM enzymes encoded by a nucleic acid sequence degenerated with respect to a nucleic acid sequence listed in any of (c) to (e).

5. The method of any of claims 1 to 4, wherein the mono-, or di-substituted 1,2,4,5-tetrazine is selected from the group consisting tetrazines, wherein the one or two carbon substituents in positions 3 and 6 are selected from the group consisting of
(a) analytical substituents, optionally for identifying and/or quantifying poly(peptides),
(b) purification substituents, optionally for at least partially purifying poly(peptides),
(c) diagnostic substituents,
(d) medicinal substituents,
(e) reactive substituents,
(f) solubilization substituents, and
(g) stabilization substituents.

6. The method of claim 5, wherein
(a) the analytical substituents are selected from the group consisting of fluorescent molecules, FRET-labels, BRET-labels, radioactive substituents, and recognition labels;
(b) the purification substituents are selected from the group consisting of biotin and peptide substituents;
(c) the diagnostic substitutents are selected from the group consisting of radiolabels and near-IR fluorescent dyes;
(d) the medicinal substitutents are selected from the group consisting of cancer chemotherapeutic agents, cellular proliferation reducing agents, antimetabolite agents, cytotoxic natural products, macrocyclic immunosuppressants, microtubule affecting agents, hormone modulators and steroids, and immunosuppressants;
(e) the reactive substituents are selected from the group consisting of alkynes, transcyclooctene, norbornene, aldehyde, tetrazine, azide, difluorooctyne, nitrones, cyclopropenes; and
(f) the solubilization substituents are selected from the group consisting of biological polymers and non-natural polymers;
(g) the stabilization substituents are selected from the group consisting of herein mentioned substituents and small organic substituents;

7. The method of any of claims 1 to 6, wherein the mono-, or di-substituted 1,2,4,5-tetrazine is a tetrazine, wherein the one or two carbon substituents in positions 3 and 6 are independently selected from the group consisting of
hydrogen, halogen, optionally CI, Br, and F, linear or branched, substituted and non-substituted alkyl, alkenyl, alkynyl, alkoxy, amino, carboxyl, carboxyl ester, acyl, acyloxy, acyl amino, amino acyl, alkylamide, sulfonyl, thioalkoxy, thioalkoxy, aryl, and heteroaryl, cycloalkyl, and heterocyclyl, (poly)peptides, (poly)peptide derivatives, peptidometics, and mono-, di-, tri- and polysaccharides.

8. The method of any of claims 1 to 7, wherein the mono-, or di-substituted 1,2,4,5-tetrazine is selected from the group consisting of tetrazines, wherein the one or two substituents in positions 3 and 6 are independently selected from the substituents listed in any of claims 5 to 7.

9. The method of any of claims 1 to 8, wherein the α-keto-β³-glycine(aminopyruvate) comprising compound is a (poly)peptide that is located within a cell expressing an rSAM enzyme, and the tetrazine compound is added extracellularly, wherein optionally the (poly)-peptide and/or the rSAM enzyme are secreted from the cell.

10. The method of claim 9, wherein the rSAM enzyme expressing cell is a natural or recombinant cell, optionally selected from
(a) naturally rSAM enzyme expressing cells, optionally *Pleurocapsa sp.,* proteobacteria, *Frankia* spp., actinomycetes, *Thiothrix* spp.;
(b) recombinant cells suitable for expressing an extrinsic rSAM enzyme are *E. coli,* optionally *E. coli* BL21, *E. coli* Rosetta, *E. coli* Tuner, *Pleurocapsa sp.* PCC 7319, proteobacteria, *Frankia* spp., actinomycetes, *Thiotrix* spp., *Pleurocapsa* sp. PCC 7327, and mammalian cell lines, optionally CHO, HeLa, and HEK cells.

11. The method of any of claims 1 to 10, wherein
(1) the compound comprising at least one α-keto-β³-amino acid (aminopyruvate) is a (poly)peptide;
(2) the 1,2,4,5-tetrazine is a mono- or disubstituted 1,2,4,5 tetrazine;
(3) the carbon substituents corresponding to carbon positions 3 and 6 on 1,2,4,5-tetrazine are selected from the substituents listed in any of claims 5 to 7;
(4) the rSAM enzyme is selected from the enzymes listed in claim 4; and/or
(5) the rSAM enzyme expressing cell is selected from the cells listed in claim 10.

12. A (poly)peptide, (poly)peptide derivative or peptidomimetic comprising at least one pyridazine, the pyridazine forming part of the (poly)peptide, (poly)peptide derivative or peptidomimetic backbone, optionally obtainable or obtained by a method according to any of claims 1 to 11.

13. The (poly)peptide, (poly)peptide derivative or peptidomimetic according to claim 12, comprising one or two analytical, purification, diagnostic, medicinal, reactive, solubilization and/or stabilization substituents bound to at least one pyridazine, optionally one or two substituents listed in any of claims 5 to 8.

14. Use of a (poly)peptide, (poly)peptide derivative or peptidomimetic according to claims 12 and 13 for compound identification, quantification, purification, stabilization and/or solubilization.

15. Use of a (poly)peptide, (poly)peptide derivative or peptidomimetic according to claims 12 and 13 for use in therapy or diagnosis.
